# EUROPÄISCHE PATENTSCHRIFT

(11) **EP 1 132 368 B1**
(45) Veröffentlichungstag und Bekanntmachung des Hinweises auf die Patenterteilung: **12.11.2003**
(21) Anmeldenummer: 01109541.1
(22) Anmeldetag: 04.01.1995
(51) Int. Cl.: C07C 57/76

(54) **Verfahren zur Herstellung von 2-Fluor-1,1-dichlor-acrylsäurechlorid**
Process for the preparation of 2-fluoro-1,1-dichloro-acrylyl chloride
Procédé pour la préparation de chlorure de 2-fluoro-1,1-dichloro-acryloyle

(30) Priorität: 17.01.1994 DE 4401099
(43) Veröffentlichungstag der Anmeldung: 12.09.2001
(62) Teilanmeldung aus: 95905118.6
(73) Patentinhaber: Bayer CropScience AG, 40789 Monheim (DE)
(72) Erfinder: Krämer, Wolfgang, Dr., 51399 Burscheid (DE); Fischer, Reiner, Dr., 40789 Monheim (DE); Holmwood, Graham, Dr., 42327 Wuppertal (DE); Hagemann, Hermann, Dr., 51375 Leverkusen (DE); Wachendorff-Neumann, Ulrike, Dr., 56566 Neuwied (DE); Erdelen, Christoph, Dr., 42799 Leichlingen (DE); Turberg, Andreas, Dr., 42781 Haan (DE); Mencke, Norbert, Dr., 51381 Leverkusen (DE)

(56) Entgegenhaltungen:
- US-A- 2 665 307
- DATABASE BEILSTEIN [Online] BEILSTEIN INSTITUTE FOR ORGANIC CHEMISTRY, FRANKFURT/MAIN, DE; Database accession no. 243008 XP002172792 & MYSKA ET AL.: COLLECT. CZECH. CHEM. COMMUN., Bd. 28, 1963, Seiten 3154-58,
- PATENT ABSTRACTS OF JAPAN vol. 006, no. 085 (C-103), 22. Mai 1982 (1982-05-22) & JP 57 018646 A (NIPPON TOKUSHU NOYAKU SEIZO KK), 30. Januar 1982 (1982-01-30)

## Beschreibung

Gegenstand der vorliegenden Erfindung ist das α-Fluor-β,β-dichlor-acrylsäurechlorid der Formel (VI)

Cl₂C = CF - COCl (VIà

seine Herstellung und Verwendung.

Aus US-A-2 665 307 und Collect. Czech. Chem. Commun. B. 28, 1963, 3154-58 ist Trichloracrylsäurechlorid bekannt.

Das α-Fluor-β,β-dichlor-acrylsäurechlorid der Formel (VI) wird erhalten, indem man 2-Fluor-1,1,3,3,3-Pentachlorpropen gegebenenfalls in Gegenwart eines Katalysators hydrolysiert. Als Katalysatoren kommen Lewis-Säuren, anorganische Säuren und deren saure Salze, wie z.B. FeCl₃, BF₃, H₂SO₄, HCI, KHSO₄, NaHSO₄ u.a. in Frage (vgl. auch die Herstellungsbeispiele).

In Abhängigkeit von den Reaktionsbedingungen (wie beispielsweise der Verweilzeit des Säurechlorids in der Reaktionsmischung) und von der zugesetzten Wassermenge wird das primär gebildete α-Fluor-β,β-dichlor-acrylsäurechlorid gegebenenfalls teilweise zur entsprechenden Acrylsäure hydrolysiert, welche anschließend beispielsweise durch Umsetzung mit Thionylchlorid wieder in das Säurechlorid überführt werden kann.

Es ist bereits bekannt, dass bestimmte Oxazolin-Derivate insektizide und akarizide Eigenschaften aufweisen (vgl. EP-A 0 345 775 und EP-A 0 432 661). Die Wirksamkeit dieser vorbekannten Verbindungen ist jedoch, insbesondere bei niedrigen Aufwandmengen und Konzentrationen nicht in allen Anwendungsgebieten völlig zufriedenstellend.

Es wurden nun die neuen substituierten Oxazoline der Formel (I) gefunden, 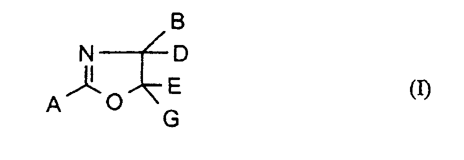 in welcher
- A: für Cl₂C=CF- steht;
- B: für jeweils gegebenenfalls substituiertes Phenyl, Phenylalkyl, Phenylalkenyl, Phenoxyalkyl oder Phenylthioalkyl steht;
- D: für Wasserstoff oder Alkyl steht;
- E: für Wasserstoff oder Alkyl steht; und
- G: für Wasserstoff oder Alkyl steht.

Die Verbindungen der Formel (I) können auch in Abhängigkeit von der Art der Substituenten als geometrische und/oder optische Isomere oder Isomerengemische unterschiedlicher Zusammensetzung vorliegen. Die Erfindung betrifft sowohl die reinen Isomeren als auch die Isomerengemische.

Weiterhin wurde gefunden, daß man substituierte Oxazoline der Formel (I) erhält, wenn man

### a) Aminoalkohole der Formel (II)

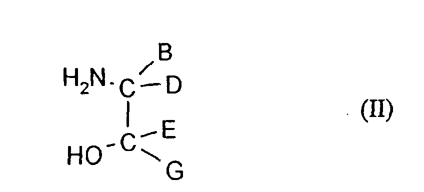

in welcher
- B, D, E und G: die oben angegebenen Bedeutungen haben,
mit einer Carbonsäure der Formel (III)

A-COOH (III)

in welcher
- A: die oben angegebene Bedeutung hat,
mit einem wasser-entziehenden Mittel und gegebenenfalls in Gegenwart eines Verdünnungsmittels umsetzt;
oder

### b) Amidalkohole der Formel (IV)

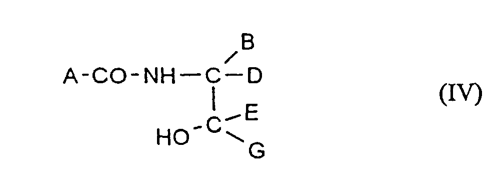

in welcher
- A, B, D, E und G: die oben angegebenen Bedeutungen haben,
mit einem wasser-entziehenden Mittel, gegebenenfalls in Gegenwart eines Verdünnungsmittels umsetzt;
oder

### c) Amid-Derivate der Formel (V)

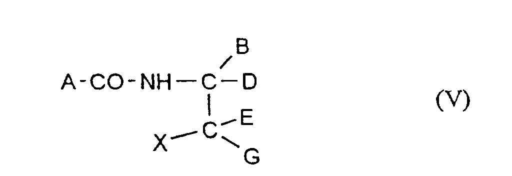

in welcher
- A, B, D, E und G: die oben angegebenen Bedeutungen haben; und
- X: für eine Abgangsgruppe, wie Halogen, Alkylsulfonyloxy oder Arylsulfonyloxy steht,
mit einer Base, gegebenenfalls in Gegenwart eines Verdünnungsmittels umsetzt.

Weiterhin wurde gefunden, dass substituierte Oxazoline der Formel (I) sehr gut zur Bekämpfung von tierischen Schädlingen geeignet sind. Sie zeichnen sich insbesondere durch hohe Wirksamkeit gegen Arthropoden und Nematoden aus.

Überraschenderweise zeigen die erfindungsgemäßen, substituierten Oxazoline der Formel (I) eine erheblich bessere Wirksamkeit gegenüber tierischen Schädlingen als die konstitutionell ähnlichsten vorbekannten Verbindungen.

Die erfindungsgemäßen Verbindungen sind durch die Formel (I) allgemein definiert.
- B: steht vorzugsweise für jeweils gegebenenfalls einfach bis vierfach, gleich oder verschieden substituiertes Phenyl, Benzyl, Pheneth-1-yl, Pheneth-2-yl, Phenoxymethyl, Phenylthiomethyl, Phenoxyeth-1-yl, Phenylthioeth-1-yl, Phenoxyeth-2-yl oder Styryl, wobei jeweils als Phenylsubstituenten genannt seien
Halogen,
C₁-C₁₈-Alkyl,
C₁-C₈-Alkoxy-C₁-C₈-alkyl,
C₁-C₈-Halogenalkoxy,
C₁-C₄-Halogenalkyl,
C₁-C₁₈-Alkoxy, das gegebenenfalls durch 1-3 weitere Sauerstoffatome unterbrochen ist,
C₁-C₁₈-Alkylthio,
C₁-C₈-Halogenalkylthio,
3,4-Difluormethylendioxo,
3,4-Tetrafluorethylendioxo,
gegebenenfalls durch C₁-C₄-Alkyl, C₃-C₆-Cycloalkyl und/oder Halogen substituiertes Benzyliminooxymethyl,
jeweils gegebenenfalls durch C₁-C₆-Alkyl, C₁-C₆-Alkoxy, Cyclohexyl oder Phenyl substituiertes Cyclohexyl und Cyclohexyloxy;
gegebenenfalls einfach oder zweifach, gleich oder verschieden durch Halogen,
C₁-C₄-Alkyl oder C₁-C₄-Halogenalkyl substituiertes Pyridyloxy;
jeweils gegebenenfalls einfach bis dreifach, gleich oder verschieden durch C₁-C₁₂-Alkyl, Halogen, C₁-C₄-Halogenalkyl, C₁-C₆-Alkoxy, C₁-C₆-Halogenalkoxy, C₁-C₆-Alkoxy-C₁-C₆-alkyl, C₁-C₆-Alkoxy-ethylenoxy, C₁-C₆-Alkylthio und/oder C₁-C₆-Halogenalkylthio
substituiertes Phenyl, Benzyl, Phenoxy, Phenylthio, Benzyloxy und Benzylthio.
- D: steht vorzugsweise für Wasserstoff oder Methyl.
- E: steht vorzugsweise für Wasserstoff oder Methyl.
- G: steht vorzugsweise für Wasserstoff oder Methyl.
- B: steht besonders bevorzugt für jeweils gegebenenfalls einfach bis vierfach, gleich oder verschieden substituiertes Phenyl, Benzyl Pheneth-1-yl, Pheneth-2-yl, Phenoxymethyl, Phenylthiomethyl, Phenoxyeth-1-yl, Phenoxyeth-2-yl,
Phenylthioeth-1-yl oder Styryl,
wobei jeweils als Phenylsubstituenten genannt seien
F, Cl Br,
C₁-C₁₈-Alkyl,
C₁-C₆-Alkoxy-C₁-C₈-alkyl,
einfach bis sechsfach, gleich oder verschieden durch F und/oder Cl substituiertes C₁-C₈-Alkoxy,
einfach bis fünffach, gleich oder verschieden durch F und/oder Cl substituiertes C₁-C₂-Alkyl,
C₁-C₁₈-Alkoxy und -(OC₂H₄)₁₋₃-O-C₁-C₆-alkyl,
C₁-C₁₂-Alkylthio,
einfach bis sechsfach, gleich oder verschieden durch F und/oder Cl substituiertes C₁-C₈-Alkylthio,
3,4-Difluormethylendioxo,
3,4-Tetrafluorethylendioxo,
die Gruppierungen 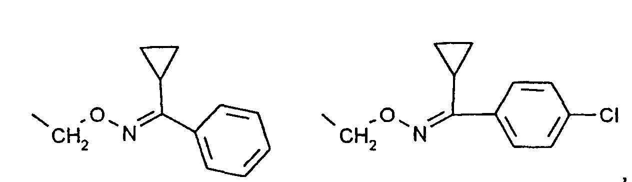 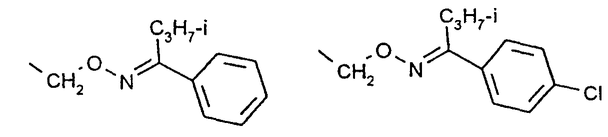 jeweils gegebenenfalls durch C₁-C₄-Alkyl, C₁-C₄-Alkoxy, Cyclohexyl oder Phenyl substituiertes Cyclohexyl und Cyclohexyloxy;
gegebenenfalls einfach oder zweifach, gleich oder verschieden durch F, Cl oder CF₃ substituiertes Pyridyloxy;
jeweils gegebenenfalls einfach bis dreifach, gleich oder verschieden durch C₁-C₁₂-Alkyl, F, Cl, Br, CF₃, C₁-C₄-Alkoxy, einfach bis sechsfach, gleich oder verschieden durch F und/oder Cl substituiertes C₁-C₄-Alkoxy, C₁-C₄-Alkoxy-C₁-C₄-alkyl, C₁-C₄-Alkoxy-ethylenoxy, C₁-C₄-Alkylthio, einfach bis sechsfach, gleich oder verschieden durch F und/oder Cl substituiertes C₁-C₄-Alkylthio
substituiertes Phenyl, Benzyl, Phenoxy, Phenylthio, Benzyloxy und Benzylthio.
- D: steht besonders bevorzugt für Wasserstoff oder Methyl.
- E: steht besonders bevorzugt für Wasserstoff oder Methyl.
- G: steht besonders bevorzugt für Wasserstoff oder Methyl.

Bevorzugte erfindungsgemäße Verbindungen sind auch Stoffgruppen der Formeln (I-1) bis (I-10): 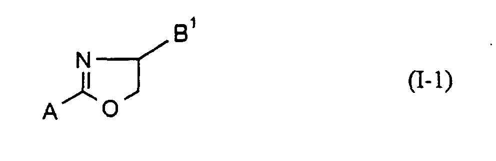 in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 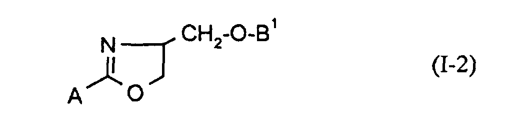
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 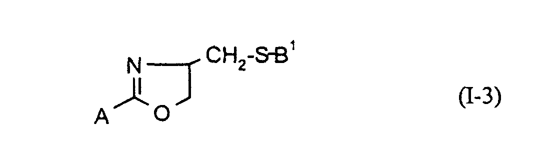
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 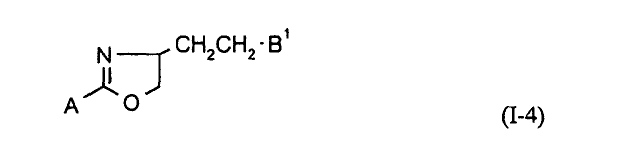
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 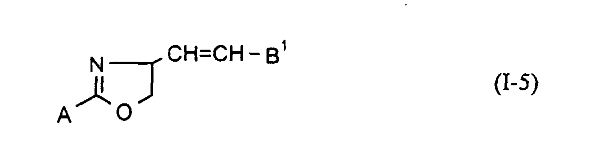
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 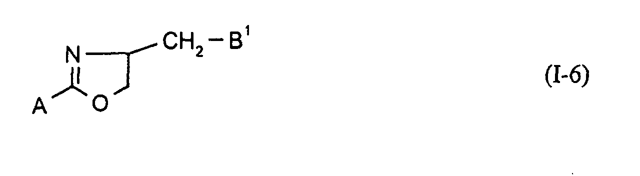
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 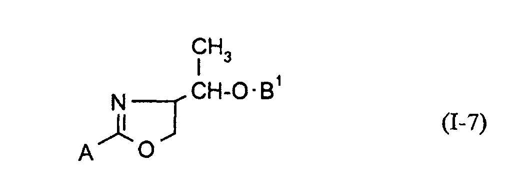
in welcher
- A: für die oben Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 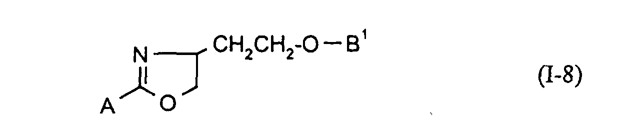
in welcher
- A: für die oben genannte Bedeutung steht und

- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 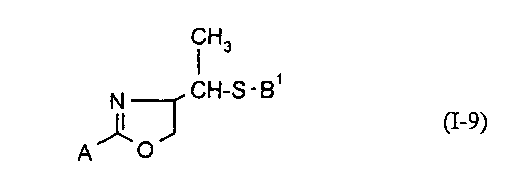
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen. 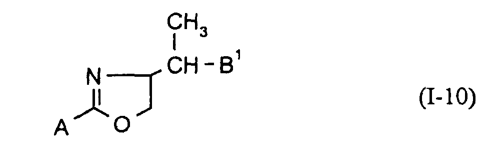
in welcher
- A: für die oben genannte Bedeutung steht und
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen.

Weiterhin bevorzugte erfindungsgemäße Verbindungen sind Stoffgruppen der Formeln (I-11) bis (I-20): 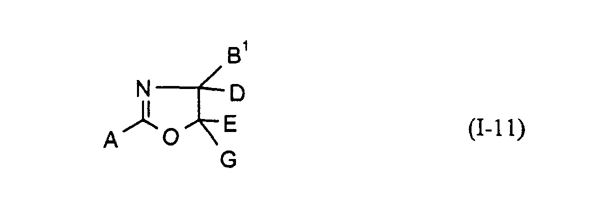 in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehen,
wobei jedoch mindestens ein Substituent für Methyl steht. 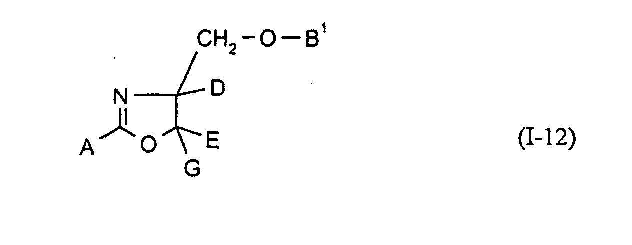
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 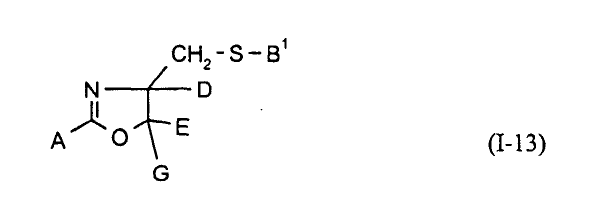
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 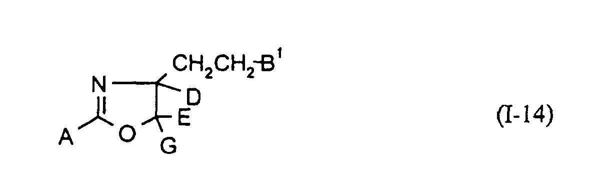
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 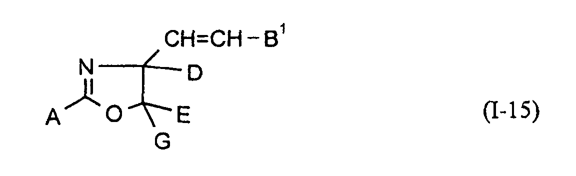
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 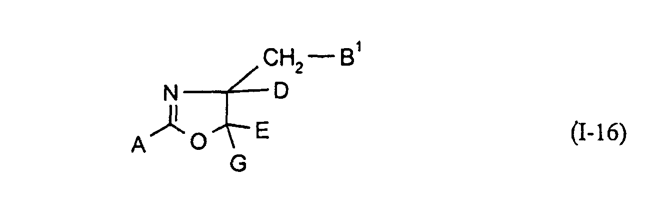
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 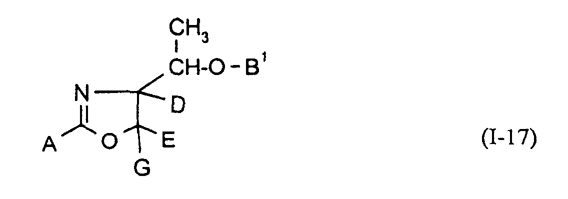
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 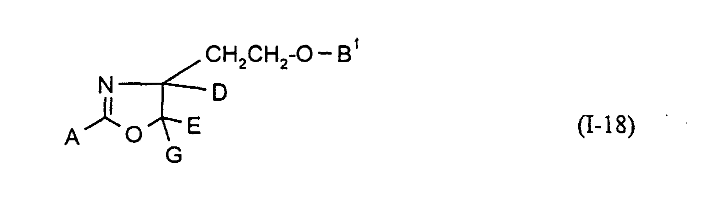
in welcher
- A: für die oben genannte Bedeutung steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 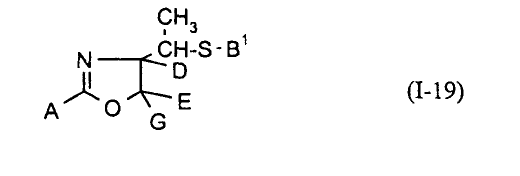
in welcher
- A: für die oben genannte Bedeutungen steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht. 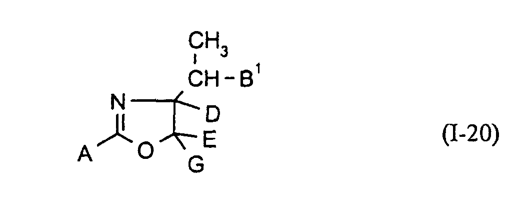
in welcher
- A: für die oben genannte Bedeutungen steht;
- B¹: für gegebenenfalls einfach bis vierfach, gleich oder verschieden substituiertes Phenyl steht, wobei als Substituenten die oben unter (B) als bevorzugt und besonders bevorzugt genannten Phenylsubstituenten in Frage kommen; und
- D, E und G: gleich oder verschieden sind und für Wasserstoff oder Methyl stehten,
wobei jedoch mindestens ein Substituent für Methyl steht.

Die oben bei der Definition der erfindungsgemäßen Verbindungen genannten Kohlenwasserstoffreste, wie Alkyl sind - auch in Verbindung mit Heteroatomen, wie Alkoxy- soweit möglich, jeweils geradkettig oder verzweigt.

Beispielhaft seien für den Substituenten B folgende Reste genannt: 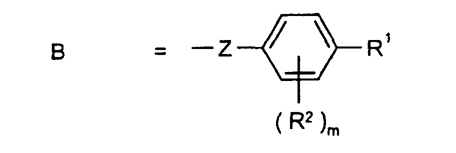 wobei
- Z =: direkte Bindung -CH₂-, -CH(CH₃)-, -CH₂CH₂-, -CH=CH-, -CH₂O-, -CH₂S-, -CH(CH₃)S-, -CH(CH₃)O-, -CH₂CH₂O-
- R¹ =: gemäß Tabelle 1 und
- (R²)ₘ =: gemäß Tabelle 2.

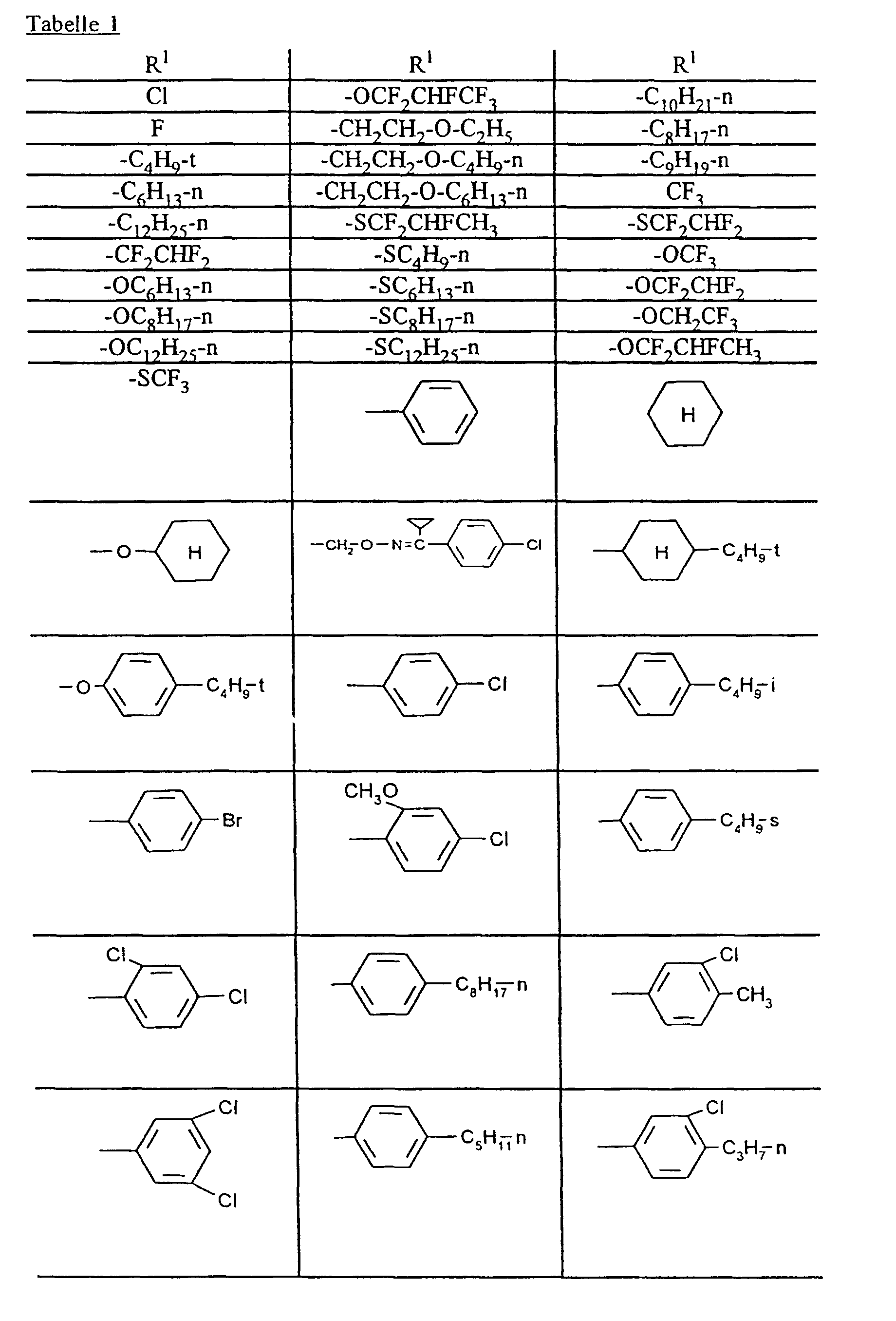

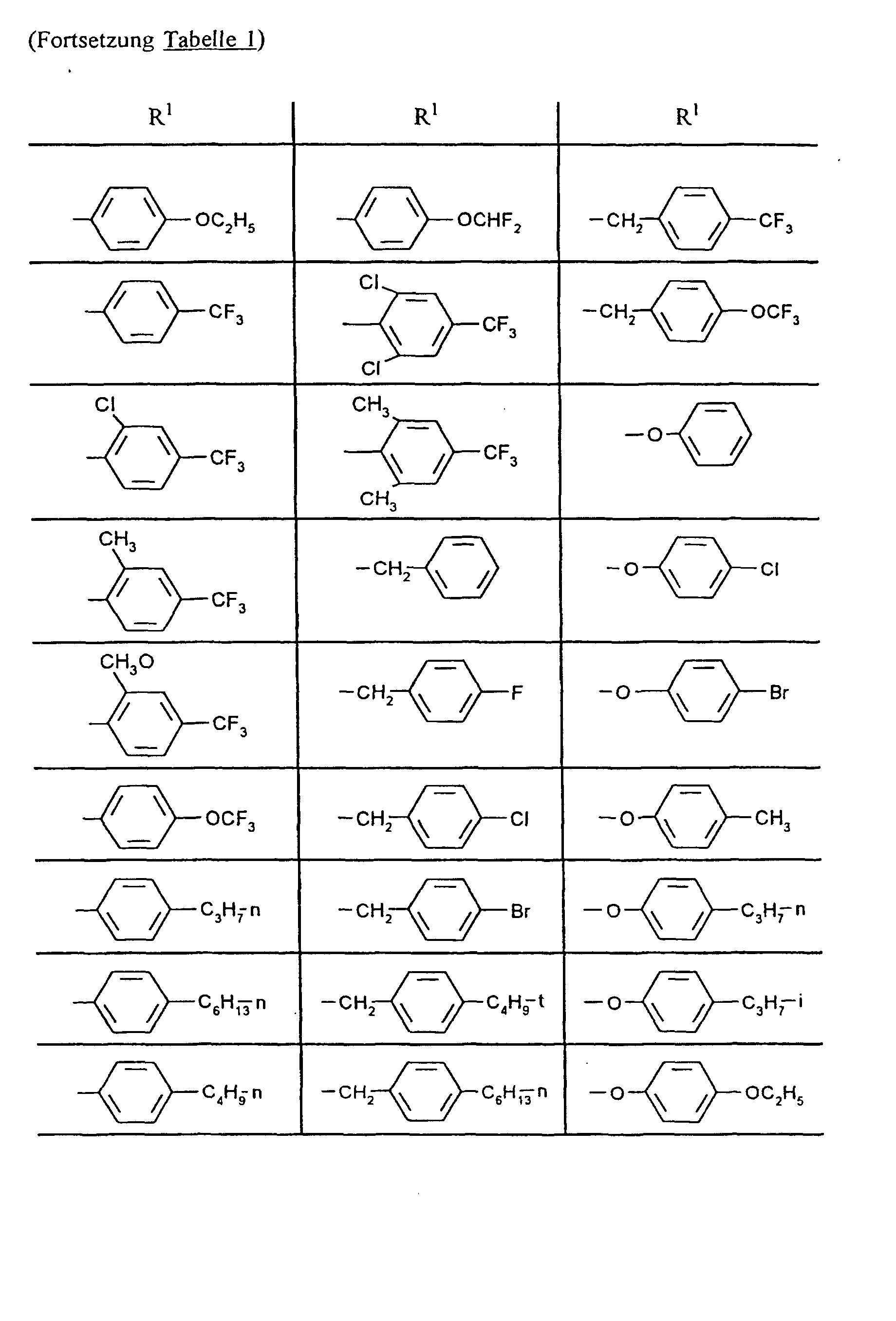

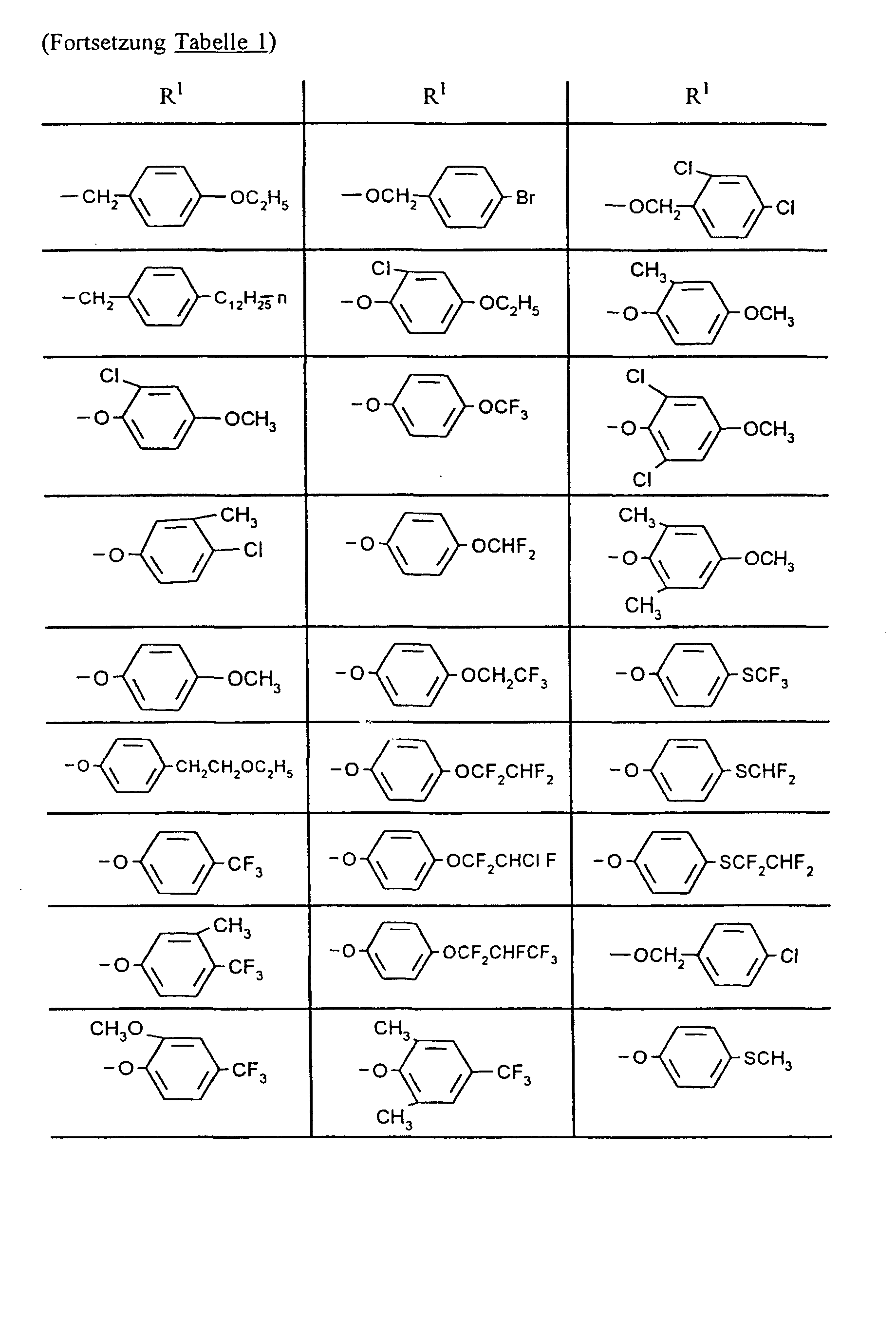

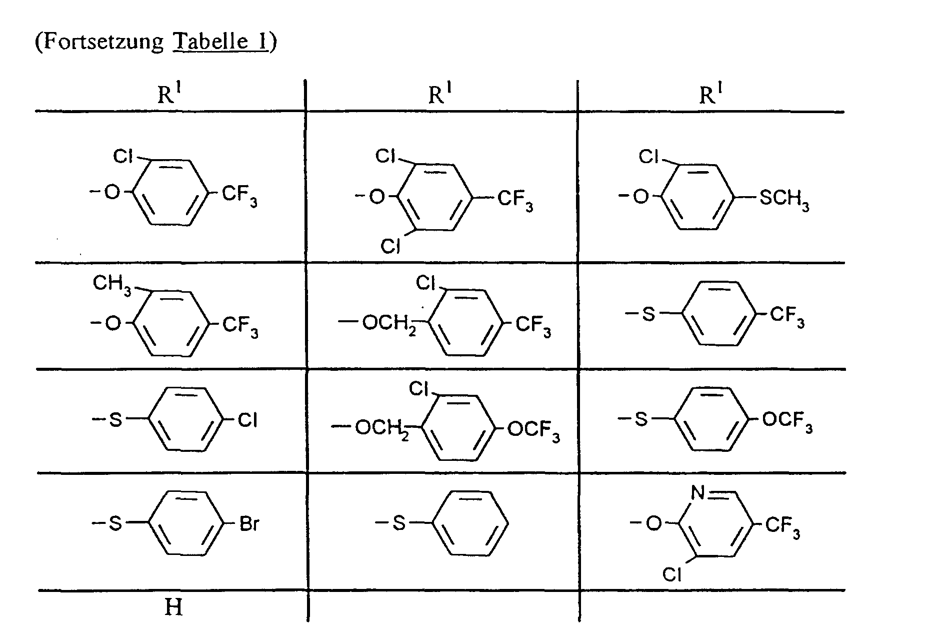

**Tabelle 2**

| (R²)ₘ | (R²)ₘ |
|---|---|
| H | 2-CH₃ |
| 2-Cl | 2-OCH₃ |
| 2-F | 2-OC₂H₅ |
| 3-Cl | 3-CH₃ |
| 2,6-Cl₂ | 3,5-(CH₃)₂ |
| 3,5-Cl₂ | 3-OC₆H₅ |
| 3,5-F₂ | |
| 2,5-Cl₂ | gemeinsam mit R¹ für |
| 3,5-Cl₂;2-F | |
| 2,3-F₂ | 3,4-OCF₂O-und |
| 2,5-F₂ | 3,4-OCF₂CF₂O- |

Im einzelnen seien folgende Beispiele für die erfindungsgemäßen Verbindungen der Formel (I) genannt:

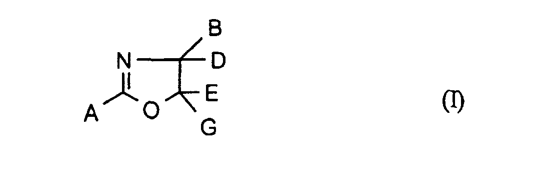

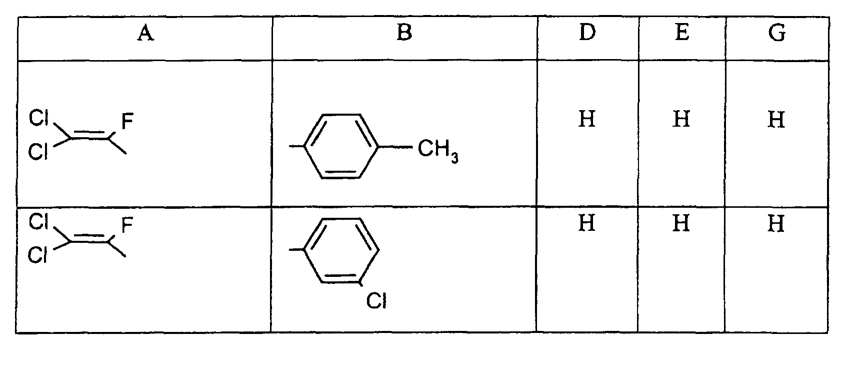

Die beim erfindungsgemäßen Verfahren (a) zur Herstellung der Verbindungen der Formel (I) weiter als Ausgangsstoffe zu verwendenden Carbonsäuren sind durch die Formel (III) allgemein definiert. In der Formel (III) hat A diejenige Bedeutung, die bereits oben im Zusammenhang mit der Beschreibung der Verbindungen der Formel (I) für A angegeben wurde.

Die Ausgangsstoffe der Formel (III) sind bekannte organischen Synthesechemikalien, bzw. in bekannter Art und Weise erhältlich.

Die erfindungsgemäßen Verfahren (a) und (b) werden unter Verwendung eines wasser-entziehenden Mittels durchgeführt. Es können die in der organischen Chemie üblichen wasser-entziehenden Mittel eingesetzt werden. Vorzugsweise verwendbar sind Schwefelsäure, Polyphosphorsäure (PPS), Phosphor(V)-oxid, Dicyclohexylcarbodiimid (DCC), Phosphor(V)-sulfid und das System Triphenylphosphin/Triethylamin/Tetrachlormethan.

Als Verdünnungsmittel zur Durchführung der erfindungsgemäßen Verfahren (a) bis (c) kommen die üblichen organischen Lösungsmittel in Betracht. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlormethan; Ether, wie Diethylether, Diisopropylether, Dioxan, Tetrahydrofuran oder Ethylenglykoldimethyl- oder -diethylether; Ketone, wie Aceton, Butanon oder Methylisobutyl-keton; Nitrile, wie Acetonitril, Propionitril oder Benzonitril; Amide, wie N,N-Dimethylformamid und N,N-Dimethylacetamid, N-Methyl-pyrrolidon oder Hexamethylphosphorsäuretriamid; Ester wie Essigsäuremethylester oder Essigsäureethylester, sowie Sulfoxide, wie Dimethylsulfoxid, gegebenenfalls auch Alkohole wie Methanol oder Ethanol.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 150°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines wasser-entziehenden Mittels durchgeführt, und das Reaktionsgemisch wird mehrere Stunden bei der jeweils erforderlichen Temperatur gerührt. Die Aufarbeitung erfolgt nach üblichen Methoden.

In einer besonderen Ausführungsform des erfindungsgemäßen Verfahrens (a) können statt der Carbonsäuren der Formel (III) auch entsprechende Nitrile eingesetzt werden, wobei dann vorzugsweise an Stelle eines wasser-entziehenden Mittels ein Katalysator, wie z.B. Zink-(II)-chlorid verwendet wird.

Die bei dem erfindungsgemäßen Verfahren (b) zur Herstellung der Verbindungen der Formel (I) als Ausgangsstoffe zu verwendenden Amidalkohole sind durch die Formel (IV) allgemein definiert. In der Formel (IV) haben A, B, D, E und G vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, B, D, E und G angegeben wurden.

Die Ausgangsstoffe der Formel (IV) sind bekannt und/oder können nach an sich bekannten Verfahren hergestellt werden.

Man erhält die Amidalkohole der Formel (IV) beispielsweise, wenn man von den Carbonsäuren der Formel (III) abgeleitete Säurechloride mit Aminoalkoholen der Formel (II) in Gegenwart eines Säurebindemittels, wie z.B. Triethylamin, Pyridin, Kaliumcarbonat, Natriumhydroxid oder Kalium-t-butylat, und in Gegenwart eines Verdünnungsmittels, wie z.B. Toluol, Chlorbenzol, Aceton oder Acetonitril, bei Temperaturen zwischen 0°C und 100°C umsetzt (vgl. die Herstellungsbeispiele).

Die von den Carbonsäuren der Formel (III) abgeleiteten Säurechloride sind weitgehend bekannt und/oder können nach an sich bekannten Verfahren, beispielsweise durch Umsetzung der Carbonsäuren der Formel (III) mit einem Halogenierungsmittel wie Thionylchlorid, gegebenenfalls in Gegenwart eines Verdünnungsmittels hergestellt werden.

Die Reaktionstemperaturen können bei der Durchführung des erfindungsgemäßen Verfahrens (b) in einem größeren Bereich variiert werden. Im Allgemeinen arbeitet man bei Temperaturen zwischen -20°C und +150°C, vorzugsweise bei Temperaturen zwischen 0°C und 100°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) zur Herstellung der Verbindungen der Formel (I) setzt man pro Mol an Amidalkohol der Formel (IV) im allgemeinen 1 bis 20 Mol, vorzugsweise 1 bis 5 Mol an wasser-entziehendem Mittel ein.

In einer bevorzugten Ausführungsform des erfindungsgemäßen Verfahrens (b) wird der Amidalkohol der Formel (IV) in einem Verdünnungsmittel vorgelegt und das wasser-entziehende Mittel wird dann eindosiert. Das Reaktionsgemisch wird bei der erforderlichen Temperatur bis zum Ende der Umsetzung gerührt und anschließend auf übliche Weise aufgearbeitet.

Die beim erfindungsgemäßen Verfahren (c) zur Herstellung der Verbindungen der Formel (I) als Ausgangsstoffe zu verwendenden Amid-Derivate sind durch die Formel (V) allgemein definiert. In der Formel (V) haben A, B, D, E und G vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, B, D, E und G angegeben wurden; X steht vorzugsweise für Fluor, Chlor, Brom, Iod, C₁-C₄-Alkyl-sulfonyloxy, Phenylsulfonyloxy oder Tolylsulfonyloxy, insbesondere für Chlor, Brom, Methylsulfonyloxy oder Tolylsulfonyloxy.

Die Ausgangsstoffe der Formel (V) sind bekannt und/oder können nach an sich bekannten Verfahren hergestellt werden.

Man erhält die Amid-Derivate der Formel (V), wenn man entsprechende Amidalkohole der Formel (IV) mit Chlorierungsmitteln, wie z.B. Thionylchlorid oder Phosphor(V)-chlorid, bzw. mit Sulfonylierungsmitteln, wie z.B. Methansulfonsäurechlorid oder p-Toluolsulfonsäurechlorid auf übliche Weise gegebenenfalls in Gegenwart einer Base und gegebenenfalls in Gegenwart eines Verdünnungsmittels umsetzt.

Das erfindungsgemäße Verfahren (c) wird in Gegenwart einer Base durchgeführt. Es kommen hierbei alle üblichen anorganischen oder organischen Basen in Betracht. Vorzugsweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +150°C, vorzugsweise bei Temperaturen zwischen 0°C und 100°C.

Das erfindungsgemäße Verfahren (c) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (c) zur Herstellung der Verbindungen der Formel (I) setzt man pro Mol an Amid-Derivat der Formel (V) im allgemeinen 1 bis 3 Mol, vorzugsweise 1,0 bis 1,5 Mol einer Base ein.

In einer bevorzugten Ausführungsform des erfindungsgemäßen Verfahrens (c) werden das Amid-Derivat der Formel (V) und eine Base in einem geeigneten Verdünnungsmittel vermischt; das Gemisch wird bei der erforderlichen Temperatur bis zum Ende der Umsetzung gerührt und anschließend auf übliche Weise aufgearbeitet.
Die Wirkstoffe eignen sich zur Bekämpfung von tierischen Schädlingen, vorzugsweise Arthropoden und Nematoden, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam.

Die erfindungsgemäßen Verbindungen zeigen in entsprechenden Aufwandmengen auch eine fungizide Wirkung.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen und in Hüllmassen für Saatgut, femer in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgae, wie Halogenkohlenwasserstoffe sowie Butan, Propan, Stickstoff und Kohlendioxid; als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgierund/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus den Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten und Endoparasiten) wie Schildzecken, Lederzecken, Räudemilben, Laufmilben, Fliegen (stechend und leckend), parasitierende Fliegenlarven, Läuse, Haarlinge, Federlinge, Flöhe und endoparasitisch lebende Würmer. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpem, wie Halsbändern, Ohrmarken, Schwanzmarken, Gliedmaßenbändern, Halftern, Markierungsvorrichtungen usw.

Die Herstellung und die Verwendung der erfindungsgemäßen Stoffe werden durch die folgenden Beispiele veranschaulicht.

### Herstellungsbeispiele

### Beispiel 2

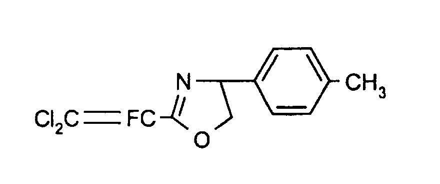

### (Verfahren c)

3,4 g (20 mmol) 2-(4-Methylphenyl)-2-aminoethanol werden in 50 ml abs. Tetrahydrofuran mit 2,8 ml Triethylamin versetzt und bei 0-10°C 3,6 g (20 mmol) 3,3-Dichlor-2-fluor-acrylsäurechlorid zugetropft. Nach einer Stunde Rühren bei Raumtemperatur wird der Niederschlag abgesaugt, das Filtrat eingeengt, der Rückstand in 50 ml abs. Toluol aufgenommen, mit 4,8 g Thionylchlorid versetzt und zwei Stunden unter Rückfluß erhitzt. Im Vakuum wird das Toluol und überschüssiges Thionylchlorid entfernt. Der Rückstand wird erneut in Toluol aufgenommen, portionsweise mit 3 g (22 mmol) Kalium-tert.-butylat versetzt und unter dünnschicht chromatographischer Kontrolle auf 50°C erwärmt. Die Reaktionsmischung wird mit Wasser gewaschen und die Toluolphase eingeengt. Der Rückstand wird an Kieselgel mit n-Hexan/Essigester 10:1 chromatographiert.

Man erhält 2,3 g (41 % der Theorie) 2-(2,2-Dichlor-1-fluorvinyl)-4-(4-methylphenyl) 1,3-oxazolin; ¹HNMR (500 MHz, CDCl₃) : 2,33 (s, 3H); 4,25 (t, 1H); 7,15 (m, 4H).

### Herstellung der Ausgangsverbindung

Es werden 515 g 2-Fluor-1,1,3,3,3-pentachlorpropen und 27 g Eisen-(III)-chlorid vorgelegt und bei 110°C innerhalb von 2 Stunden 52,6 g Wasser zugetropft. Man läßt 1 Stunde bei 110°C nachrühren, kühlt auf ca. 70°C ab, trennt durch Filtration die Feststoffe ab und tropft 330,2 ml Thionylchlorid zu. Nach ca. 2 Stunden Rückfluß werden das überschüssige Thionylchlorid und das Dichlorfluoracrylsäurechlorid destillativ getrennt.

Man erhält 233 g (90 % der Theorie) Dichlorfluoracrylsäurechlorid vom Siedepunkt 134°C.

## Patentansprüche

1. Verbindung der Formel (VI)
CCl₂=CF-COCl (VI)

2. Verfahren zur Herstellung von der Verbindung der Formel (VI) gemäß Anspruch 1, **dadurch gekennzeichnet, dass** man 2-Fluor-1,1,3,3,3,-Pentachlorpropen gegebenenfalls in Gegenwart eines Katalysators hydrolysiert.

3. Verfahren zur Herstellung der Verbindung der Formel (VI) gemäß Anspruch 2, **dadurch gekennzeichnet**, das man Lewis-Säuren als Katalysatoren verwendet.

4. Verwendung von Verbindung der Formel (VI) gemäß Anspruch 1 zur Herstellung von Verbindungen der Formel (I) 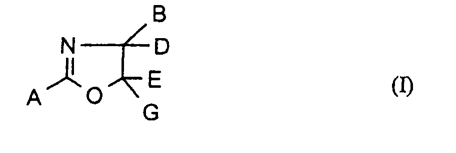 in welcher
A für Cl₂C=CF- steht;
B für jeweils gegebenenfalls substituiertes Phenyl, Phenylalkyl, Phenylalkenyl, Phenoxyalkyl oder Phenylthioalkyl steht;
D für Wasserstoff oder Alkyl steht;
E für Wasserstoff oder Alkyl steht; und
G für Wasserstoff oder Alkyl steht.

## Claims

1. Compound of the formula (VI)
CCl₂=CF-COCl (VI)

2. Process for the preparation of the compound of the formula (VI) according to Claim 1, **characterized in that** 2-fluoro-1,1,3,3,3-pentachloropropene is hydrolysed optionally in the presence of a catalyst.

3. Process for the preparation of the compound of the formula (VI) according to Claim 2, **characterized in that** Lewis acids are used as catalysts.

4. Use of compound of the formula (VI) according to Claim 1 for the preparation of compounds of the formula (I) 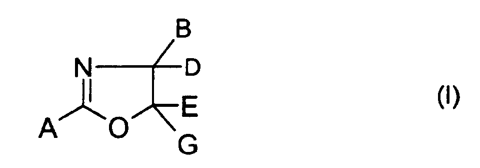 in which
A represents Cl₂C=CF-;
B represents in each case optionally substituted phenyl, phenylalkyl, phenylalkenyl, phenoxyalkyl or phenylthioalkyl;
D represents hydrogen or alkyl;
E represents hydrogen or alkyl; and
G represents hydrogen or alkyl.

## Revendications

1. Composé de la formule (VI) :
Cl₂C = CF - COCl (VI)

2. Procédé de préparation du composé de la formule (VI) suivant la revendication 1, **caractérisé en ce que** l'on hydrolyse le 2-fluoro-1,1,3,3,3-pentachloropropène en présence d'un catalyseur.

3. Procédé de préparation du composé de la formule (VI) suivant la revendication 2, **caractérisé en ce que** l'on utilise des acides de Lewis comme catalyseur.

4. Utilisation du composé de la formule (VI) suivant la revendication 1, pour la préparation de composés de la formule (I) : 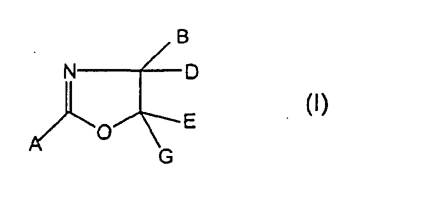 dans laquelle :
A représente Cl₂C=CF- ;
B représente un radical phényle, phénylalcoyle, phénylalcényle, phénoxyalcoyle ou phénylthioalcoyle, le cas échéant substitué ;
D représente l'atome d'hydrogène ou un radical alcoyle ;
E représente l'atome d'hydrogène ou un radical alcoyle, et
G représente l'atome d'hydrogène ou un radical alcoyle.
